# EUROPÄISCHE PATENTSCHRIFT

(11) **EP 0 892 635 B1**
(45) Veröffentlichungstag und Bekanntmachung des Hinweises auf die Patenterteilung: **27.09.2000**
(21) Anmeldenummer: 97954421.0
(22) Anmeldetag: 18.12.1997
(51) Int. Cl.: A61K 7/48, A61K 7/06

(54) **KOSMETISCHE ZUBEREITUNGEN ENTHALTEND EXTRAKTE VON PHYLLANTHUS EMBLICA UND CENTELLA ASIATICA UND/ODER BACOPA MONNIERI**
COSMETIC PREPARATIONS CONTAINING EXTRACTS FROM PHYLLANTUS EMBLICA AND CENTELLA ASIATICA AND/OR BACOPA MONNIERI
PREPARATIONS COSMETIQUES CONTENANT DES EXTRAITS DE PHYLLANTHUS EMBLICA ET DE CENTELLA ASIATICA ET/OU DE BACOPA MONNIERI

(30) Priorität: 28.12.1996 DE 19654635
(43) Veröffentlichungstag der Anmeldung: 27.01.1999
(73) Patentinhaber: Singh-Verma, Shyam Bir, Dr., D-50169 Kerpen (DE)
(72) Erfinder: Singh-Verma, Shyam Bir, Dr., D-50169 Kerpen (DE)
(74) Vertreter: Jönsson, Hans-Peter, Dr.Dipl.-Chem.
(86) Internationale Anmeldenummer: EP9707113
(87) Internationale Veröffentlichungsnummer: WO9829089

(56) Entgegenhaltungen:
- EP-A- 0 345 571
- FR-A- 1 433 383
- GB-A- 2 274 058
- PATENT ABSTRACTS OF JAPAN vol. 015, no. 206 (C-0835), 27.Mai 1991 & JP 03 058939 A (TOREEDE UINDO KK), 14.März 1991,
- J. F. MORTON: "The Emblic (Phyllantus emblica L:)" ECONOMIC BOTANY, Bd. 14, 1960, Seiten 119-128, XP002063499 in der Anmeldung erwähnt
- F. D'AMELIO: "Gotu Kola" COSMETICS & TOILETRIES, Bd. 102, Nr. 6, 1987, NY (USA), Seiten 49-50, XP002063500

## Beschreibung

Gegenstand der Erfindung sind kosmetische Zubereitungen zur topischen Applikation enthaltend Extrakte von Phyllanthus emblica und Centella asiatica und/oder Bacopa monnieri sowie die Verwendung dieser Zubereitungen zur Pflege der menschlichen Haut.

Gemäß dem deutschen Lebensmittel- und Bedarfsgegenstandsgesetz (LMBG), 4. Abschnitt vom 15. August 1974, der EG-Richtlinie (76/768/EWG) vom 27. September 1976 und der deutschen Kosmetik-Verordnung vom 26. Juni 1985 sind kosmetische Mittel Stoffe oder Zubereitungen aus Stoffen, die dazu bestimmt sind, äußerlich am Menschen oder in seiner Mundhöhle unter anderem zur Reinigung, Pflege und Schutz angewendet zu werden. Bei der Festlegung dieser Definition wurde dem Gedanken des Verbraucherschutzes Priorität eingeräumt.

Lange Zeit wurde insbesondere von Dermatologen die Auffassung vertreten, daß durch die Barriereschicht der Haut außer Wasser nur wenige Stoffe durchgelassen werden und die kosmetischen Mittel somit keine Wirkung besitzen oder auch sollten. In der Zwischenzeit konnte belegt werden, daß eine Vielzahl von Stoffen in Abhängigkeit ihres Molekulargewichtes, ihrer Molekülstruktur und -größe auch durch die intakte und gesunde Haut penetrieren können. Wenn man sich zusätzlich besonderer Trägersubstanzen, insbesondere Emulgatoren bedient, erhöht sich die Anzahl um ein Vielfaches.

Der Verbraucher erwartet von kosmetischen Mitteln auch spezifische Wirkung wie beispielsweise die Vorbeugung gegen vorzeitige Hautalterung, die Vermeidung oder zeitliche Verzögerung von Fältchenbildung oder einen effektiveren Schutz gegen die Austrocknung der Haut. Dabei wird vorausgesetzt, daß die eingesetzten Inhaltsstoffe frei von schädlichen Einflüssen sind.

Diese Verbrauchererwartung führte zur Entwicklung sogenannter "Wirkkosmetik", wobei die eingesetzten Wirkstoffe jedoch keine systemische Wirkung besitzen dürfen. Es kamen insbesondere Vitamine, Mineralstoffe und Spurenelemente oder tierische Proteine zum Einsatz.

Hingegen bieten sich im Bereich der Heilpflanzen erhebliche Möglichkeiten die oben genannten Effekte ohne nennenswerte Gesundheitsrisiken zu erzielen, da ihre Verträglichkeit aus der Überlieferung der Volksheilkunde in allen bedeutenden Kulturen hinreichend belegt ist. Darauf beruht insbesondere die Wirkung der Phytopharmaka, wobei in vielen Fällen auch die Wirkstoffe aus Pflanzen als Reinsubstanzen zum Einsatz gelangt sind. Die Wirkung von Pflanzenextrakten ist häufig schwächer als die der Reinsubstanzen. Teilweise wirken aber auch die Bestandteile eines Extraktes nur als Ganzes. Bei chronischen Erkrankungen bieten Phytopharmaka eine echte Alternative mit geringeren Gesundheitsrisiken zu den allopathischen Heilmitteln.

In der 5. Auflage (1995) von "International Cosmetic Ingredient Dictionary", herausgegeben von dem amerikanischen Industrieverband CTFA, sind etwa 300 Pflanzen aufgelistet, die in unterschiedlichen Zubereitungen als Wirkstoffe den kosmetischen Mitteln zugesetzt werden. Eine weniger umfangreiche Liste "Einsatz von pflanzlichen Wirkstoffen und Extrakten in der Hautpflege" wurde von D. Bilek, S. B. Singh-Verma und P. Bernhardt veröffentlicht (1989), SÖFW 115, Jg.Nr. 19, S. 331-338.

EP 0 345 571 B1 betrifft eine pflanzliche Wirkstoffzubereitung zur Verwendung in Kosmetika, die insbesondere eine Tinktur, hergestellt aus luftgetrockneten Früchten der Amlapflanze (Phyllanthus emblica) zum Inhalt hat. Bei der Amlafrucht handelt es sich um eine indische Heilpflanze, deren medizinische Eigenschaften in "Economic Botany" 14/1960, S. 119-128 ausführlich beschrieben worden sind. Die Tinktur aus Amlafrucht enthält hauptsächlich in hohen Konzentrationen entzündungshemmende Gerbstoffe, Schleimsäure, verschiedene Fruchtzucker und eine Reihe von freien Aminosäuren sowie ein von Natur aus stabilisiertes Vitamin C. Wie in umfangreichen Anwendungstests mit amlatinkturhaltigen Haut- und Haarpflegemitteln nachgewiesen werden konnte, eignet sich dieses Stoffgemisch als kosmetischer Wirkstoff gut.

Als weitere Heilpflanze von Bedeutung für die Kosmetikindustrie ist Gotu Kola (Brahmi) zu nennen. Diese Bezeichnung umfaßt zwei Spezies in wechselnden Anteilen nämlich "Centella asiatica" und "Bacopa monnieri", die je nach Verwendungszweck einzeln oder auch in Kombination zum Einsatz kommen. Als wirksame Inhaltsstoffe einer nach HRBI hergestellten Urtinktur werden das Alkaloid Hydrocotylin, die Triterpensäuren (Asiatsäure, Madecass-Säure, Madasiatsäure) und das Triterpensaponin Asiaticosid genannt (Haager's Handbuch der Drogenkunde). Der oralen Applikation eines Aufgusses der Heilpflanze werden blutreinigende, tonisierende und diuretische Eigenschaften nachgesagt. Bei topischer Anwendung besitzen die Extrakte und Tinkturen antiphlogistische, antibakterielle und wundheilende Wirkung, die auf die Beeinflussung der, in den Fibroblasten stattfindenden Bildung von Kollagenfasern, das heißt erhöhte Kollagenbildung bei verzögerter Vernarbung zurückzuführen sein soll.

Aus Hager's Handbuch der Pharmazeutischen Praxis Springer Verlag, 1972, 3. Band, S. 792-793 ist bekannt, daß der Wirkstoff Madecassoid antiinflammatorisch wirkt, während das Mitosen anregende Asiaticosid heilungsfördernd bei Premitis und Wunden wirkt.

Bekanntlich findet bei jugendlichen Erwachsenen eine Erneuerung der Hornschicht in einem Zyklus von ca. drei Wochen statt. Mit zunehmendem Alter verlangsamt sich diese Proliferationsrate, beispielsweise bei 50-jährigen und älteren Menschen in erheblichem Maße. Dies bedeutet, daß die Haut sich nicht in drei-, sonder in fünfoder gar sechswöchigem Zyklus erneuert mit der Folge, daß sie dünner und unter dem Einfluß schädlicher Umweltbedingungen auch trockener wird. Das Ergebnis ist sichtbare Faltenbildung im Gesichts- und Körperbereich.

Demgegenüber besteht die Aufgabe der vorliegenden Erfindung in der Bereitstellung von kosmetischen Zubereitungen zur topischen Applikation zur Pflege der menschlichen Haut, insbesondere zur Beschleunigung der natürlichen Hautregeneration.

Die vorgenannte Aufgabe wird in einer ersten Ausführungsform der Erfindung gelöst durch kosmetische Zubereitungen zur topischen Applikation enthaltend Extrakte von Phyllanthus emblica und Centella asiatica und/oder Bacopa monnieri neben an sich bekannten Hilfs- und Trägerstoffen.

Es wurde überraschenderweise gefunden, daß die Kombination der beiden Extrakte beim Einsatz als Wirkstoff in Haut- und Körperpflegepräparaten eine besondere kosmetische und pflegende Eigenschaft bewirken, die dazu führt, daß die natürliche Hautregeneration beschleunigt wird.

Neben den beiden obengenannten Extrakten sieht die Erfindung in bevorzugten Ausführungsformen den Einsatz von zusätzlichem Aloe barbadensis und Hibiscus sabdariffa als Emollient mit feuchtigkeitsspendenden oder feuchtigkeitsbindenden und hautglättenden Eigenschaften vor.

Die Erfindung geht dabei davon aus, daß die Inhaltsstoffe der Extrakte so als Wirkstoff zur Verfügung stehen, daß eine rasche Einwirkung auf der Hautoberfläche gegeben ist. Hierzu ist es von besonderer Bedeutung, die Inhaltsstoffe möglichst schonend und vollständig aus den Pflanzenbestandteilen der Amlapflanze beziehungsweise der Gotu Kola zu extrahieren. Das anzuwendende Extraktionsverfahren richtet sich wesentlich nach der Art des herzustellenden Pflegemittels, für das der betreffende Extrakt eingesetzt werden soll. Die Inhaltsstoffe der Amlapflanze haben bekanntermaßen eine adstringierende, entfettende und - bei längerer Anwendung - auf die Tätigkeit der Talgdrüsen positiv beeinflussende und deutlich regulierende Wirkung. Bei Gesichts- und Hautpflegepräparaten für fette oder Problemhaut ergibt sich bei höheren Einsatzkonzentrationen eine eindeutige kosmetische Wirkung. Die Haut wird durch die Anwendung eines entsprechenden Extraktes bereits gestrafft und bekommt ein glattes und geschmeidiges Aussehen ohne Mitesser und sonstige Hautunreinheiten. Bei niedriger Dosierung ergibt sich bekanntermaßen für eine trockene und Mischhaut eine ausgezeichnete tonisierende und regenerierende Wirkung. Ethanolische Extrakte, insbesondere ethanolische Extrakte aus Früchten sind als Hautpflegemittel bei fettender aber auch bei trockener und Mischhaut (in niedriger Dosierung) von besonderer Bedeutung.

Ein "Extrakt" im Sinne der vorliegenden Erfindung umfaßt den mit Hilfe eines Lösungsmittels gewonnenen Auszug aus den genannten Pflanzenbestandteilen, die gegebenenfalls unter Einsatz von Druck und/oder Temperatur aus den Bestandteilen gewonnen werden. Durch das einzusetzende Extraktionsmittel beziehungsweise das eingesetzte Extraktionsverfahren kann die Wirkstoffzusammensetzung nach Art und Menge geringfügig variieren.

Von besonderer Bedeutung sind Extrakte aus Früchten, die sowohl in frischer Form als auch in getrockneter Form verarbeitet werden können (Amla). In ähnlicher Weise kann der Extrakt beziehungsweise der ölige Auszug bei Centella asiatica auch aus Stengeln, Blüten, Blättern oder Wurzeln gewonnen werden. In einer bevorzugten Ausführungsform der vorliegenden Erfindung enthalten die Zubereitungen 0,1 bis 40 Gew.-% der Extrakte, insbesondere 1 bis 20 Gew.-% der Extrakte von Phyllanthus emblica und 1 bis 20 Gew.-% Centella asiatica und/oder Bacopa monnieri, wobei sich die Gewichtsangabe bei der Kombination aus Centella asiatica und Bacopa monnieri auf die Gesamtmenge der beiden Bestandteile bezieht. Wird die Einsatzkonzentration zu niedrig gewählt, so ist die gewünschte Wirkung nicht vorhanden. Werden jedoch die Extraktmengen zu hoch eingestellt, so ist eine wirtschaftliche Verwertung nicht möglich. Darüber hinaus ergeben sich erhebliche galenische Probleme bei der Verarbeitung von Extrakten in den kosmetischen Zubereitungen.

Neben den genannten Extrakten enthaltend die kosmetischen Zubereitungen, gegebenenfalls an sich bekannte Hilfs- und Trägerstoffe, wie beispielsweise aus Sucker, Fuchs und Speiser, "Pharmazeutische Technologie" (1978); Schrader, "Grundlage und Rezepturen der Kosmetika" (1989) und Nowak, "Die kosmetischen Präparate", 2. Aufl. (1975) bekannt sind. Besonders hervorzuheben sind an dieser Stelle pflanzliche Öle, Alkohole, Emulgatoren, Antioxidationsmittel, Verdickungsmittel, UV-Absorber, Vitamine, Mineralstoffe, Spurenelemente und/oder Parfum. Eine besonders bevorzugte Zubereitungsform ist beispielsweise ein Fluid, das ausschließlich natürliche Wirkstoffe und die genannten Pflanzenextrakte enthält. Diese regen die Blutzirkulation an und fördern die Regeneration der Haut. Natürliche Öle gleichen den Fett- und Feuchtigkeitshaushalt der Haut aus und verleihen ihr Elastizität und Spannkraft. Vitamine stimulieren die Zellaktivität und schützen vor Umwelteinflüssen. Bevorzugt ist ein entsprechendes Fluid frei von Farb- und Konservierungsstoffen, zieht schnell in die Haut ein und fettet nicht. Weitere Zubereitungsformen sind Öl in Wasser-(O/W)-Emulsionen beispielsweise Feuchtigkeitsfluide oder Nachtcremes.

Eine weitere Ausführungsform der vorliegenden Erfindung betrifft die Verwendung von Zubereitungen wie oben definiert zur Pflege der menschlichen Haut, insbesondere zur Beschleunigung der natürlichen Hautregenerierung.

Da der deutsche Gesetzgeber die Durchführung von Tierversuchen für die Entwicklung kosmetischer Mittel verbietet, war es nicht möglich die Proliferation der Epidermis direkt nachzuweisen. Es wurden daher in mehreren Stufen unter streng kontrollierten Bedingungen Anwendungstests durchgeführt werden, um eine synergistische Wirkung der Kombination von Extrakten aus Phyllanthus emblica und Centella asiatica und/oder Bacopa monnieri nachzuweisen.

Deshalb wurden aus der Gesamtdroge luftgetrocknete Früchte von Phyllanthus emblica (Amla) Urtinkturen (Extrakte) nach HRB I (Extraktionsmittel 70 % Ethanol und 30 % demin. Wasser) hergestellt und als Wirkstoffe für kosmetische Mittel verwendet (Amla-Tinktur).

### Ausführungsbeispiele

### Vergleichsbeispiel 1:

In streng kontrollierten Probandengruppen von 15 Personen wurden folgende Trägergrundlagen miteinander verglichen:
- Schüttelemulsion:
   bestehend aus natürlichen Triglyceriden wie zum Beispiel Saflor-, Süßmandel-, Aprikosenkern- und Sesamöl in einer Gesamtmenge von 79 Gew.-%, 8 Gew.-% Ethanol, 10 Gew.-% Pflegestoffe und Vitamine wie Aloe barbadensis, Hibiscus sabdariffa, Algengel, Eibisch - und Kamilleblütenextrakte, Vitamin-A-Palmitat, Vitamin-E-Acetat, 1 Gew.-% Hilfsstoffe wie Seidenproteine, Lecithin, Antioxidationsmittel, Parfum sowie 2 Gew.-% Amlatinktur;
- Öl in Wasser Emulsionen auf Basis nicht-ionogener Emulgatoren:
   1.) Fettphase, bestehend aus dem Emulgator Triceteareth-40 Phosphat in einer Menge von 5 Gew.-%, einer Fettkomponente einschließlich natürlicher Öle und Antioxidationsmittel in einer Menge von 7,5 Gew.-%, einem UV-Filter in einer Menge von 1,5 Gew.-%,
      Wasserphase in einer Menge von 74 Gew.-% Wasser einschließlich Konservierungsmittel und Verdickungsmittel,
      Pflegestoffe und Vitamine in einer Menge von 10 Gew.-% wie Allantoin, Propylenglykol, Vitamin A-Palmitat, Vitamin E-Acetat, Aloe barbadensis, Hibiscus sabdariffa und Parfum sowie 2 Gew.-% Amlatinktur.
   2.) Fettphase, bestehend aus dem Emulgatorgemisch Glycerylstearat und PEG-100 Stearat in einer Menge von 5 Gew.-%, Bienenwachs, Lanette N und Isopropylpalmitat in einer Gesamtmenge von 7 Gew.-%, eine Fettkomponente einschließlich natürlicher Öle und Antioxidantien in einer Menge von 25 Gew.-%,
      Wasserphase in einer Menge von 50 Gew.-%,
      Pflegestoffe und Vitamine in einer Menge von 11 Gew.-% wie Butylenglykol, Allantoin, Algenextrakt, D-Panthenol, Vitamin A-Palmitat, Vitamin E-Acetat und Parfum sowie Amlatinktur in einer Menge von 2 Gew.-%.

In den beiden Trägersystemen Schüttelemulsion und den beiden O/W Creme wurde der Wirkstoff "Amla-Tinktur" eingearbeitet. Es wurden pro Trägersystem zwei Laboransätze mit und ohne Amla-Tinktur hergestellt. Die Schüttelemulsionen wurden in 30 ml Glasflaschen und die Creme in 50 ml Tuben abgefüllt und mit unterschiedlichen Chargen-Nrn. versehen.

Es wurden drei Gruppen zu jeweils fünf Probanden gebildet, um alle drei Trägersysteme in einem Versuchsdurchgang zu testen. Bei den Probanden handelte es sich um überwiegend weibliche Personen im Alter zwischen 40 und 58 Jahren. Jede Probandengruppe erhielt die entsprechenden Produktmuster mit und ohne Wirkstoffzusatz. Die wirkstoffhaltige Zubereitung und die entsprechenden Placebo wurden morgens und abend nach üblicher Gesichtsreinigung auf jeweils eine Gesichtshälfte aufgetragen. Die Dauer des Anwendungsversuches betrug sechs Wochen. Beurteilt wurden zweimal wöchentlich Glätte und Elastizität als Maßstab für die Regeneration der Haut sowie das subjektive Empfinden zum Beispiel Hautspannung, Trockenheitsgefühl und ähnliches durch die Probanden selbst.

Nach dreiwöchiger Anwendungsdauer stellte sich heraus, daß das Trägersystem Schüttelemulsion im Vergleich zu den beiden O/W-Cremes ein deutlich besseres Ergebnis nicht nur in Bezug auf die Beurteilungskriterien Hautglätte und Elastizität, sondern auch in der subjektiven Beurteilung durch die Verwender erbrachte. Darüber hinaus wurde im Gegensatz zu handelsüblichen Cremes das schnelle Einziehen der natürlichen Öle in die Haut ohne unangenehme Klebeeffekte, sowie die "convenience" bei der Anwendung positiv beurteilt. Zwischen den beiden O/W-Cremes untereinander gab es keinen nennenswerten Unterschied.

Hingegen waren Unterschiede zwischen dem Placebo und der wirkstoffhaltigen Formulierung bei allen drei Trägersystemen festzustellen. Dieser Effekt war bei dem Trägersystem "Schüttelemulsion" am stärksten ausgeprägt und wurde auch subjektiv von allen Probanden bestätigt.

### Vergleichsbeispiel 2:

Nach den obengenannten Ergebnissen wurden Versuche für das Trägersystem "Schüttelemulsion" mit analogen Zusammensetzungen durchgeführt, wobei jedoch die Urtinkturen von Phyllanthus emblica und ein 50 % zu 50 % Gemisch aus Centella asiatica und Bacopa monnieri miteinander verglichen. Die Urtinkturen beider Heilpflanzen wurden in obiger Schüttelemulsionsgrundlage in einer Konzentration von jeweils 5 Gew.-% eingearbeitet und dementsprechend die Menge der Ölphase reduziert.

Die beiden Formulierungen wurden an einer Probandengruppe von 10 Personen wiederum in halbseitigem Anwendungstest auf ihre Wirksamkeit miteinander vergleichen. Bei den Probanden handelte es sich um überwiegend weibliche Personen im Alter zwischen 45 und 60 Jahren. Die Versuchsdauer betrug sechs Wochen.

Die Produkte wurden nach üblicher Gesichtsreinigung morgens und abends halbseitig aufgetragen. Neben der üblichen Beurteilung auf Hautglätte und Elastizität sowie das subjektive Empfinden zum Beispiel Hautspannung, Trockenheitsgefühl und ähnliches durch die Probanden wurden noch zwei weitere Parameter hinzugezogen. Ein signifikantes Zeichen der Proliferation der Epidermis (Neubildung der Hautzellen) ist das Abstoßen der toten Zellen der Hornschicht. Da sich die neugebildete Hautschicht durch Glätte und frische Farbe auszeichnet, wurde die Tiefe der Fältchen im Augen-, Mund- und Stirnbereich ebenfalls mitbewertet. Die Beurteilungsintervalle wurden von zwei- auf dreimal wöchentlich erhöht.

Bei 60 % der Probanden wurde eine deutliche Abschuppung der Hornschicht nach dreiwöchiger Anwendungsdauer festgestellt. Nach einer weiteren Woche der Anwendung wurden gleiche Beobachtungen auch bei den restlichen 40 % der Probanden, die über 55 Jahre alt waren, gemacht. Am Ende des Anwendungstests nach 6 Wochen waren bei allen Probanden die kleinen Fältchen im Gesicht fast verschwunden und selbst tieferen Fältchen deutlich abgeflacht. Damit konnte der Beweis für die Proliferation der Epidermis erbracht werden.

Zwischen den beiden Urtinkturen von Phyllanthus emblica und Centella asiatica und Bacopa monnieri konnten graduell Unterschiede zu Gunsten der ersteren beobachtet werden. Es zeigte sich, daß die mit Amla-tinkturhaltigen Schüttelemulsionen behandelte Gesichtshälfte glatter und frischer aussah und eine Regeneration der Haut um ein paar Tage früher festzustellen war. Aufgrund der Inhaltsstoffe der Amlatinktur (Schleimsäuren, Gerbstoffe, Fruchtzucker, freie Aminosäuren, Vitamin C und andere) wie bereits eingangs beschrieben, war dieses Ergebnis erwartungsgemäß.

Neben der Normalisierung bzw. Förderung natürlicher Regeneration (Zellproliferation) alter, reifer Haut, eignet sich die erfindungsgemäße Schüttelemulsion zur Behandlung von Akne vulgaris, Pubertätsakne und sonstigen Hautunreinheiten, wie Mitesser und ähnliches ebenfalls besonders. Diese Wirkung ist vermutlich auf zwei Faktoren zurückzuführen:
- Wegen ihrer unterschiedlichen Spreitwirkung dängen die natürlichen Triglyceride (Öle) in unterschiedlichen Tiefen der Hautschichten und erreichen somit auch die Talgdrüsen. Sie lösen den Talg auf und ermöglichen somit ein freies Ausfließen an die Hautoberfläche. Damit wird ein Verstopfen der Talgdrüsen und Kanäle verhindert.
- Die Heilpflanzenextrakte und Vitamine werden von den natürlichen Ölen ebenfalls in tiefere Hautschichten transprotiert. Sie bewirken eine Normalisierung der Zellaktivitäten und auch eine antiphlogistische Wirkung.

Es konnte festgestellt werden, daß ein gezielter Zusatz von

| | |
|---|---|
| Aloe barbadensis (10faches Konzentrat) | 3 bis 5 Gew.-% |
| Hibiscus Sabdariffa (Blütenextrakt) | 2 bis 6 Gew.-% |
| Algengel | 3 bis 6 Gew.-% |

zu der Schüttelemulsion als auch zu den O/W- und W/O-Emulsionen die Pflegeeigenschaften (wie z.B. Glätte, Weichheit, Feuchtigkeitsspende) dieser Produkte in erheblichen Maße verbessert.

### Beispiel 1:

Nach einer Versuchspause von mehreren Monaten wurde bei den selben Probanden ein weiterer Versuch zur Feststellung der synergistischen Wirkung der Kombination von Phyllanthus emblica und ein 50 % zu 50 % Gemisch aus Centella asiatica und Bacopa monnieri durchgeführt. Zu diesem Zweck wurde die Urtinkturen von Phyllanthus emblica und ein 50 % zu 50 % Gemisch aus Centella asiatica und Bacopa monnieri in einer Konzentration von jeweils nur 2 Gew.-% in obige Schüttelemulsionsgrundlage eingearbeitet.

Alle andere Versuchsbedingungen sowie die Beurteilungsparameter bleiben unverändert.

Überraschenderweise wurde festgestellt, daß bereits nach zweiwöchiger Anwendungsdauer bei etwa 75 % der Probanden eine deutliche Abschuppung der Hornschicht eingetreten war. Die gleiche Erfahrung machten auch die restlichen 25% der Probanden nach 3 Wochen, so daß zusätzlich die Anwendung eines handelsüblichen Gesichts-Peeling-Creme empfohlen wurde, um die abgestorbenen Hautzellen der Hornschicht abzutragen. Der Altersunterschied der Probanden spielte dabei keine wesentliche Rolle.

Dieses Ergebnis zeigt die synergistische Wirkung der Urtinkturen von Phyllanthus emblica und ein 50 % zu 50 % Gemisch aus Centella asiatica und Bacopa monnieri, denn es konnte eine bessere Proliferation der Epidermis in kürzerer Zeit und mit niedrigerer Konzentration als bei den Einzeltinkturen erzielt werden. Das Ergebnis bereits nach einer 3-wöchigen Anwendung der oben beschriebenen Schüttelemulsion war eine glatte, elastische und frisch aussehende Haut mit deutlich und objektiv wahrnehmbar reduzierten Fältchen im Gesicht. Selbst wenn die Anwendung auf einmal täglich über Nacht reduziert wurde, ließ sich die Haut in einem attraktiv und gesund wirkenden Zustand über längere Zeit hinweg aufrechterhalten. Da sich die Durchführung diese Anwendungstests über fast zwei Jahre erstreckte, ließen sich jahreszeitbedingte Schwankungen ausschließen.

### Beispiel 2:

Im weiteren Verlauf der Untersuchungen wurden auch die O/W Cremeformulierungen auf der Basis nicht-ionogener Emulgatoren mit der Wirkstoffkombination aus Urtinkturen von Phyllanthus emblica und ein 50 % zu 50 % Gemisch aus Centella asiatica und Bacopa monnieri in einer Menge von 2 Gew.-% Amla und 3 Gew.-%, ein 50 % zu 50 % Gemisch aus Centella asiatica und Bacopa monnieri versetzt und ebenfalls auf ihre hautregenerierende Eigenschaften geprüft. Auch bei diesen Formulierungen konnte ein vergleichbares Ergebnis erzielt werden.

### Beispiel 3:

### Kosmetische Zubereitung gegen nicht genetisch bedingten Haarausfall und Haarpflegemittel

In der Praxisanwendungserfahrung konnte nachgewiesen werden, daß der Einsatz von Phyllanthus emblica und Centella asiatica und/ocer Bacopa monnieri in geeigneten Konzentrationen und Trägersystemen bei älterer und reifer Haut zur Normalisierung der Zellproliferation (Regeneration der Haut) führt.

Es werden bei der Anwendung der erfindungsgemäßen Wirkstoffkombination gemäß Beispiel 2 überraschenderweise auch die Aktivität der Kopfhautzellen durch den Einsatz von Phyllanthus emblica, Bocapa monnieri und Centella asiatica sowie noch zusätzlichen Heilpflanzen normalisiert bzw. stimuliert. Dafür wurden eine Vielzahl von Heilpflanzenextrakte getestet. Folgende Pflanzenextrakte wiesen eine stimulierende Wirkung auf und wurden zu einem Wirkstoffkomplex verarbeitet:

Wirkstoffkomplex gegen Haarausfall und Stimulation des Haarwachstums

| | |
|---|---|
| Phyllantus emblica | 25 Teile |
| Bacopa monnieri | 10 Teile |
| Centella asiatica | 10 Teile |
| Abies balsamaea (Weißtannenzapfenöl) | 20 Teile |
| Mentha arvensis, piperascens (Öl) | 10 Teile |
| Hibiscus sabdariffa (Blütenextrakt) | 10 Teile |
| Melia azadirachta (Blätterextrakt) | 5 Teile |
| Citrus bergamia (Bergamotöl) | 5 Teile |
| Citrus aurantifolia (Limonenblütenöl | 5 Teile |
| Gesamt: | 100 % |

Auf Basis dieser Wirkstoffkombination wurden folgende kosmetische Formulierungen gegen nicht genetisch bedingten Haarausfall und Förderung des normalen Haarwachstums hergestellt:

| Medizinisches Haar-Tonikum | | |
|---|---|---|
| | | Vorzugsformulierung |
| Wirkstoffkombination gemäß Beispiel 1 | 1 - 50 % | 15 % |
| Pflegestoffe und Vitamine (I-Menthol, D-Panthenol, Camphor, β-Estradiol, Nicotinsäureamid) | 0,5 - 3,0 % | 1,5 % |
| Hilfsmittel - PEG-40-Hydrogenated Castor Oil - Solvent Ethoxydiglycol | 0,3 - 1,5 % | 0,5 % |
| Parfumöl | 0,3 - 1,0 % | 0,5 % |
| Isopropanol | 20,0 % | 20,0 % |
| Ethanol | ad 100 % | 62,5 % |
| Gesamt: | | 100 % |

| Haarkur (Emulsionsform) | | |
|---|---|---|
| | | Vorzugsformulierung |
| Cetylstearylalkohol | 0,5 - 2,0 % | 0,5 % |
| nicht-ionogener Emulgator | 2,5 - 5,0 % | 3,5 % |
| Ölkomponenten (wie bei Schüttelemulsion + Melia azadirachta Kernöl) | 3,0 - 25,0 % | 20,0 % |
| Wirkstoffkombination gemäß Beispiel 1 | 2,0 - 30 % | 20,0 % |
| Glycerin 86 %ig | 1 - 5 % | 3,0 % |
| Citronensäure | 0,1 - 0,5 % | 0,5 % |
| demin. Wasser | ad 100 % | 52,5 % |
| Gesamt: | | 100 % |

### Versuchsbeispiel:

In den bisherigenVersuchen (Dauer ca. 3 Monate) wurden die Produkte wie folgt angewandt:
- Haarkur: Zweimal pro Woche

| | |
|---|---|
| Einwirkungsdauer mindestens | 30 Minuten |
| vorzugsweise | 60 Minuten |

- Haar-Tonikum: täglich morgens und abends mindestens aber 1 x täglich und stets nach dem Haarewaschen.
Das Haartonikum durfte nicht abgewaschen werden und verblieb auf dem Kopf. Es wurde leicht mit kreisenden Fingerspitzen-Bewegungen in die Kopfhaut einmassiert.

Ergebnis:
- Nach ca. 10wöchiger Anwendung war eine signifikante Reduzierung des Haarausfalls festzustellen.
- Das Wachstum der vorhandenen Haare wurde ebenfalls deutlich gefördert.

Um die Behandlung der Kopfhaut zu fördern und Haarausfall zu reduzieren wurden drei milde Shampoo-Formulierungen entwickelt und zwar für:
- normales und feines Haar
- fettiges und schuppiges Haar
- trockenes Haar.

Als Beispiel wird hier eine Shampoo-Formulierung für normales und feines Haar angegeben:

| | |
|---|---|
| Mischung aus milden waschaktiven Substanzen | 40 % |
| Pflegestoffe und Avivagemittel (natürliche Triglyceride z.B. Süßmandel bzw. Aprikosenkernöl, Guar-Hydroxypropyltriammonium, Chlorid und Rückfetter) | ( 0,5 - 2 %) vorzugsweise 1,0 % |
| Wirkstoffkombination gemäß Beispiel 1 | (1 - 8 %) vorzugsweise 3,0 % |
| Konservierungsmittel-Lösung | 1,0 % |
| Parfumöl | 0,2 % |
| Demin. Wasser | 54,8 % |
| Gesamt: | 100 % |

## Patentansprüche

1. Kosmetische Zubereitungen zur topischen Applikation enthaltend Extrakte von Phyllanthus emblica und Centella asiatica und/oder Bacopa monnieri neben an sich bekannten Hilfs- und Trägerstoffen.

2. Zubereitungen nach Anspruch 1, enthaltend einen Extrakt aus frischen und/oder getrockneten Früchten der Phyllanthus emblica Linné.

3. Zubereitungen nach Anspruch 1, enthaltend einen aus der Gesamtdroge, insbesondere Stengel, Blüten, Blättern und Wurzeln gewonnenen Extrakt der Centella asiatica und/oder Bacopa monnieri.

4. Zubereitungen nach Anspruch 1, enthaltend 1 bis 20 Gew.-% des Extraktes von Phyllantus emblica und 1 bis 20 Gew.-% Centella asiatica und/oder Bacopa monnieri.

5. Zubereitungen nach einem der Ansprüche 1 bis 4, dadurch gekennzeichnet, daß die Hilfs- und Trägerstoffe ausgewählt sind aus pflanzlichen Ölen, Alkohol, Emulgatoren, Antioxidantien, Verdickungsmitteln, UV-Absorbern, Vitaminen, Mineralstoffen, Spurenelementen und/oder Parfum.

6. Zubereitungen nach einem der Ansprüche 1 bis 5, enthaltend Extrakte von Aloe barbadensis und Hibiscus sabdariffa.

7. Verwendung von Zubereitungen nach einem der Ansprüche 1 bis 6 zur Pflege der menschlichen Haut, insbesondere zur Beschleunigung der Proliferation der Epidermis sowie zur Behandlung von nicht genetisch bedingtem Haarausfall.

## Claims

1. Cosmetic formulations for topical application containing extracts from *Phyllanthus emblica* and *Centella asiatica* and/or Bacopa *monnieri* in addition to per se known adjuvants and carriers.

2. The formulations according to claim 1, containing an extract from fresh and/or dried fruits of *Phyllanthus emblica* Linné.

3. The formulations according to claim 1, containing an extract from *Centella asiatica* and/or *Bacopa monnieri* obtained from the full drug, especially from the stalks, flowers, leaves and roots.

4. The formulations according to claim 1, containing from 1 to 20% by weight of said extract from *Phyllanthus emblica* and from 1 to 20% by weight of *Centella asiatica* and/or *Bacopa monnieri.*

5. The formulations according to any of claims 1 to 4, characterized in that said adjuvants and carriers are selected from vegetable oils, alcohols, emulsifiers, antioxidants, thickeners, UV absorbers, vitamins, minerals, trace elements and/or perfume.

6. The formulations according to any of claims 1 to 5, containing extracts from *Aloe barbadensis* and *Hibiscus sabdariffa.*

7. Use of formulations according to any of claims 1 to 6 for the care of the human skin, especially for accelerating epidermic proliferation and for the treatment of non-genetically caused loss of hair.

## Revendications

1. Préparations cosmétiques pour l'application locale contenant des extraits de Phyllanthus emblica et de Centella asiatica et/ou de Bacopa monnieri outre des adjuvants et supports connus.

2. Préparations selon la revendication 1, contenant un extrait de fruits frais et/ou séchés de Phyllanthus emblica Linné.

3. Préparations selon la revendication 1, contenant un extrait de Centella asiatica et/ou de Bacopa monnieri obtenu à partir de l'ensemble de la drogue, en particulier les tiges, les fleurs, les feuilles et les racines.

4. Préparations selon la revendication 1, contenant 1 à 20 % en poids de l'extrait de Phyllanthus emblica et 1 à 20 % en poids de Centella asiatica et/ou Bacopa monnieri.

5. Préparations selon l'une des revendications 1 à 4, caractérisées en ce que les adjuvants et supports sont choisis parmi les huiles végétales, les alcools, les émulsifiants, les antioxydants, les épaississants, les absorbants d'UV, les vitamines, les matières minérales, les oligo-éléments et/ou les parfums.

6. Préparations selon l'une des revendications 1 à 5, contenant des extraits d'Aloe barbadensis et d'Ibiscus sabdariffa.

7. Utilisation de préparations selon l'une des revendications 1 à 6, pour le soin de la peau humaine, en particulier pour accélérer la prolifération de l'épiderme ainsi que pour le traitement de la chute de cheveux d'origine non génétique.
